# EUROPEAN PATENT SPECIFICATION

(11) **EP 1 064 238 B1**
(45) Date of publication and mention of the grant of the patent: **18.12.2002**
(21) Application number: 99910937.4
(22) Date of filing: 19.03.1999
(51) Int. Cl.: C04B 35/584, C04B 35/00, C04B 35/64, A61L 27/00

(54) **COMPOSITION, MANUFACTURING, AND USE OF SILICON NITRIDE AS A BIOMATERIAL FOR MEDICAL PURPOSE**
ZUSAMMENSETZUNG, HERSTELLUNG UND VERWENDUNG VON SILICIUMNITRID ALS BIOMATERIAL FÜR MEDIZINISCHE ZWECKE
COMPOSITION, FABRICATION ET UTILISATION DE NITRURE DE SILICIUM EN TANT QUE MATERIAU BIOLOGIQUE A USAGE MEDICAL

(30) Priority: 19.03.1998 SE 9800918
(43) Date of publication of application: 03.01.2001
(73) Proprietor: Biomat System AB, 111 21 Stockholm (SE)
(72) Inventor: OLSSON,Kent, S-118 67 Stockholm (SE); JIANGUO, Li, S-141 31 Huddinge (SE); LINDGREN, Urban, S-167 76 Bromma (SE)
(74) Representative: Larsson, Karin
(86) International application number: SE9900443
(87) International publication number: WO99047471

(56) References cited:
- EP-A1- 0 374 957
- WO-A1-89/03371
- WO-A1-90/11979
- GB-A- 2 025 238
- US-A- 4 617 024
- US-A- 4 957 509

## Description

### TECHNICAL AREA

The present invention refers to the composition, manufacturing, utilisation and function of silicon nitride for medical purposes, for example as a biomaterial for wearing surfaces in artificial joints.

Patients with joints, which are destroyed from fractures or degenerative joint disease, suffer from pain from the involved joint, e.g. the hip joint. In order to relieve the pain it is necessary, in addition to using pain relievers, to surgically change the involved joint. Another alternative is to replace the involved joint with an artificial joint. where the joint surfaces are manufactured from a biomaterial. The biomaterial must be durable against wear, since a joint is heavily loaded. It is necessary to choose a biomaterial, which fulfils these demands, so that a lifetime of the prosthesis can exceed the life expectancy of the patient. Today about 10% of hip prosthesis must be replaced within ten years of the implantation due to wear and loosening caused by particles which originate from the joint surfaces. It is difficult to find new biomaterials with the necessary characteristics for implantation in humans.

Ceramic materials or substances, and in particular structural ceramic materials, are generally considered to be highly resistant against corrosion and erosion. This is true for many oxides, nitrides, carbides and borides. The materials are completely inactive, that is they do not cause a reaction either indirectly or directly from the surrounding tissue, which makes them suitable as orthopaedic implants.

Silicon nitride is such a ceramic substance. Silicon nitride and mixtures in the form of powder can be pressed under high temperature and high pressure, so called isostatic pressing (HIP), to a preshaped porous body consisting of the material of the powder.

Silicon nitride is also a ceramic material which we have shown has extraordinary characteristics as a biological material and is extremely well tolerated by the biological organism. In addition the material has a high coefficient of wear, which means that it has a high wear resistance. Compared to other ceramics (see the table below) silicon nitride has excellent characteristics as a prosthetic material for implantation.

The invention as defined in claim 1 describes a mode of usage of such a ceramic substance or its mixtures as a biomaterial in a biological environment.

More specifically the invention can be defined as a way to use such a ceramic substance in the form of silicon nitride as a basis, and its mixtures, as a supporting material/wear surface e.g. in prostheses to be used for biological implantation in humans and/or animals.

Biomaterial means in this context a material or a substance, which can be sustained by biological tissues without causing a pronounced rejection.

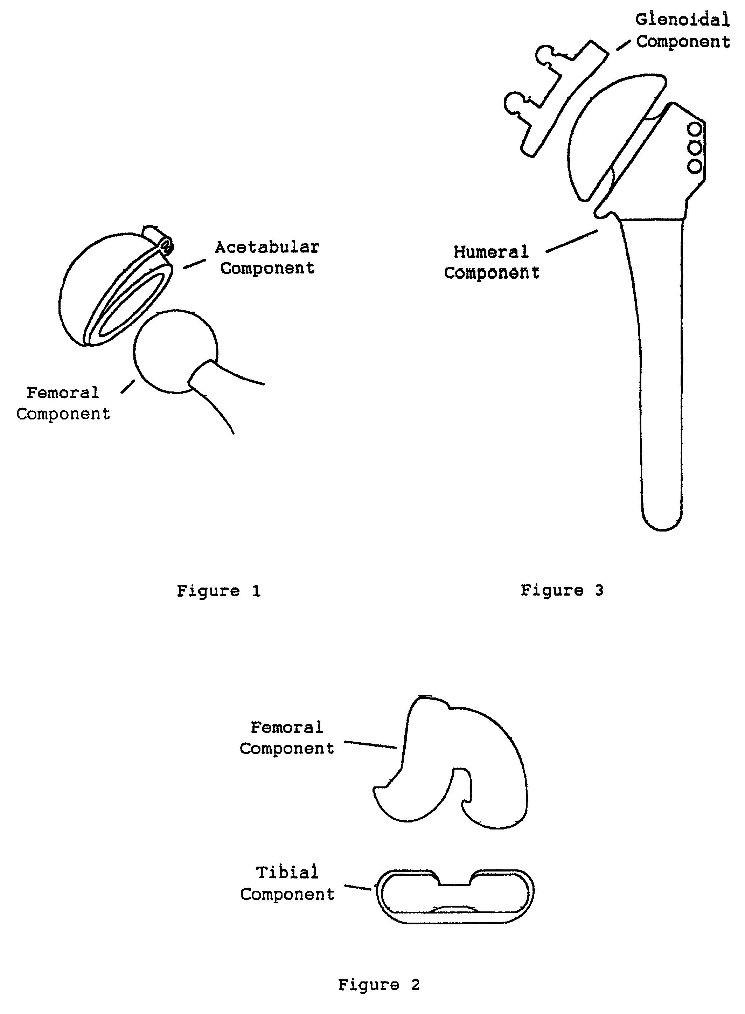

A supporting material means a material, which functions as a joint surface in an artificial joint for example in the hip joint or the knee joint. see figure 1 and 2 below.

Biological usage means that the prosthesis is surgically implanted in or carried by a biological organism.

### THE PRESENT STATE OF THE ART

When using silicon nitride as a biomaterial in the tissues of biological organisms rejection is of a minor degree without importance, that is the biological organism sustains the foreign material well and the material can therefore be used as an implant.

Silicon nitride is shaped by isostatic pressing under high temperature and high pressure to a joint surface. e.g. the joint surfaces of the hip and the knee. In order to acquire a sufficient quality of the wearing surface it is necessary that certain biological demands are met.

Through the Swedish patent application 90.04134-4 (KN 7334 SE) it is known how to perform isostatic pressing of ceramic materials to the highest degree of pore quality.

We refer to the following publications:
GB 2025238;
T. Mukarami and N. Ohtsuki. Bioceramics. Ishiyaku Euroamerica, 224-46, 1989:
P. Kumar et al. Adv. In Bio.Eng. 9, 373-8, 1990;
H. Oonishi et al., Bioceramics. Ishiyaku Euroamerica, 272-7, 1989;
H. McKellop et al., JBMR 15. 619-53, 1981:
B. Weightman and D. Light. Biomaterials 7, 20-9, 1986;
L. Zichner. Trans. Soc. Biomaterials Imp. Retrieval Symp., p.22, 1988.

### DESCRIPTION OF THE INVENTION

The purpose of the invention is to solve such problems, which have been described above involving prosthesis loosening in biological organisms, including the human. as a result of increased wear at the joint surfaces of the mechanical prosthetic joint.

According to the invention silicon nitride is used which, compared to other ceramics, is wear resistant and has excellent characteristics for implantation in the form of prosthesis. as demonstrated in the following table:

**TABLE**

| Characteristics | Alumina | Zirconia | Silicon nitride |
|---|---|---|---|
| Bending strength (MPa) | 600 | 1000 | 900 |
| Fracture toughness (MPa/m²) | 3-4 | >8 | 4.5 |
| Hardness (GPa) | 22 | 12 | 19 |

The joint surfaces are manufactured from silicon nitride and/or its mixtures by isostatic pressing under high temperatures, high pressures and sintering, at which:
1. Silicon nitride as a base and its mixtures (see below) creates the prerequisites for a wear resistant material which is suitable for joint bearing components in prostheses for biological organisms, including man.
2. The powder material from which the object is formed/manufactured consists of a ceramic material. silicon nitride. but it can also be composed of intermetallic compounds or composites consisting of mixtures of metallic and ceramic materials,
3. One, both or all joint surfaces in the same artificial joint are manufactured of the mixture according to the example below.
4. The minimal porosity of the joint surface achieved by using HIP for its manufacturing, limits the wear.
5. The polished surface contributes to decreased wear.
6.The specific microstructure of the joint surface increases the liquid film between the joint surfaces in order to limit wear by increased lubrication from the body fluid.
7. The processing of the joint surface leads to a decreased risk for the formation of cavities or crevices.
8. The angular movement of the joint influences the wear, and manufacturing of small components e.g. a hip with a small prosthetic head (ball) which can be 22 mm or less in diameter from silicon nitride and/or mixtures of the biomaterials mentioned below.
9. Special methods of desinfection avoid a risk of infection for the biological organism.

The invention is of particular value for the manufacturing of surfaces in artificial joints, for which good surface characteristics, such as hardness and wear resistance, is a necessary requirement, and where surface defects such as irregularities, crevices and cracks can not be tolerated.

Examples of products with such requirements are joint surfaces. i.e. both the prosthetic head (ball) and the so called cup (socket), in hip prostheses, joint surfaces, i.e. both the thigh bone (femoral) and the shin bone (tibial) component, in knee prosthesis and joint surfaces, e.g. in the shoulder, the ankle and so forth, where the characteristics of the joint surface is decisive, and where silicon nitride and/or mixtures of the biomaterials mentioned below make up the prosthetic body.

### FIGURE TEXT

One presently suggestive variant of the invention will be described below with reference to drawings included where
figure 1 shows the wearing surfaces in an artificial hip joint,
figure 2 shows the wearing surfaces in an artificial knee joint,
figure 3 shows the wearing surfaces in an artificial shoulder joint,
figure 4 shows the components of an artificial hip joint. and
figure 5 shows the components in an artificial knee joint.

### DETAILED MANUFACTURING DESCRIPTION

Below are a couple of examples of suitable material compositions and material characteristics. Example 1 shows silicon nitride mixed with 2 volume % yttrium dioxide which results in the following data:

| Example 1 | |
|---|---|
| Characteristics | Value |
| Bending strength (3 pt.) | 1000 MPa |
| Weibull modulus (m) | 22 |
| Fracture toughness | 4.5 MPa/m² |
| Stress exponent | >100 |
| Hardness | 19 GPa |

Example 2 shows silicon nitride mixed with 10 volume % of aluminium oxide in the form of short fibres, which results in the following data:

| Example 2 | |
|---|---|
| Characteristics | Value |
| Bending strength (3 pt.) | 930 Mpa |
| Weibull-modulus (m) | 23 |
| Fracture toughness | 6.5 MPa/m² |
| Stress exponent | >100 |
| Hardness | 20 GPa |

Manufacturing of the material is performed by preparing a powder mixture according to one of the examples above with silicon nitride as a base or the addition of other biomaterials, which have the desired characteristics. The mixture is formed to a body and sintered under high pressure (greater than 50 MPa, in particular pressures higher than 150 MPa, but not higher than 350 MPa) and high temperature (500-3000 °C) according to the HIP-method. The HIP-method is the suitable method. since the porosity is a decisive factor for wear e.g. of the joint surfaces. After cooling the surfaces are polished, cleaned and disinfected before medical use. When the material has been consolidated, there is no chemical reaction.

For the wear at the joint surfaces of the mechanical joint to be used for implantation to be minimised a special method of polish of medical materials which fulfils class III is necessary.

In connection with the figures below. some examples of the usage of the biomaterial in connection with different artificial joints are shown.

Figure 1 demonstrates an example of a hip prosthetic head and its socket, where the joint surfaces are manufactured from silicon nitride or its mixtures according to one of the examples above. The ball has a diameter of 22 mm, but can be larger or smaller. The ball is spherical with a conical fitting to be attached to the neck of the prosthetic body. The construction is suitable for artificial hip joints in general.

Figure 2 demonstrates a knee prosthesis consisting of a thigh bone (femur) and shin bone (tibia) component which are manufactured of silicon nitride or its mixtures and which is attached to the thigh bone and the shin bone respectively. The sizes vary according to the patient's weight and size. The construction is well suited for artificial knee prostheses in general.

Figure 3 shows an example of a shoulder prosthesis in the form of a prosthetic ball and its socket, which are manufactured from silicon nitride or its mixtures.

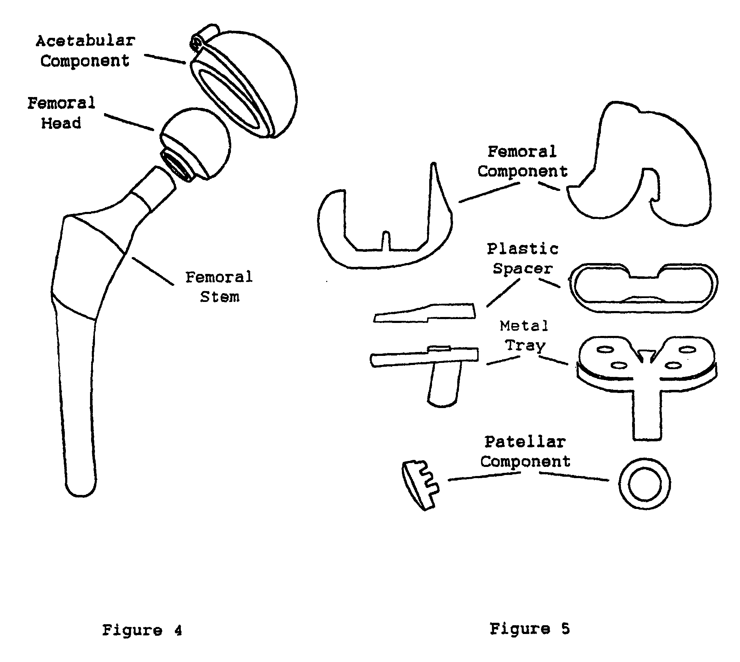

Figure 4 shows the assembly of the components in an artificial hip joint consisting of a shaft, ball and a socket, the joint surface carrying components being composed of silicon nitride or its mixtures.

A corresponding overview, from the side and form the front, of an artificial knee joint is shown in figure 5. Also in this case the joint surface carrying components, that is the thigh bone and the shin bone components, are composed of silicon nitride or its mixtures.

The invention is not limited to the designs described above which can be varied within the framework of the following patent claims. Thus one, both or all wearing surfaces in an artificial joint can be composed of one or many materials according to the invention. The invention is neither limited to the hip, the knee nor the shoulder, but can also be used for other types of joints such as elbow, ankle, finger and other possible joints.

## Claims

1. A method for the manufacturing of a joint prosthesis, for example a hip or knee joint proshesis, of a ceramic material with a high stress tolerance and tissue tolerance for medical usage as a surface carrying, orthopaedic biomaterial for the wear surfaces in the artificial joint prosthesis, **characterised by**
1.1. the preparation/mixture of a powder composition containing silicon nitride as a base consisting of 70-100 volume % silicon nitride with addition of 0-30 volume % of one or the combination of the following materials: zirconium dioxide, yttrium dioxide, titanium dioxide, calcium oxide, silicon carbide and/or 0-20 volume % of short fibres of aluminium oxide, silicon carbide and mullite, or independent mixtures of these,
1.2. shaping the joint prosthesis body from the mixture,
1.3. subjecting the prosthesis body to high pressures between 50 MPa and 350 MPa and temperatures between 500-3000 °C by isostatic pressing and sintering (HIP) in order to acquire a desirable porosity of the body, whereupon
1.4. the body is cooled and the wear surfaces are polished for the wear to be minimized and decontaminated clinically before medical usage.

2. A method according to claim 1 **characterised by** the shaping of the silicon nitride and its mixtures to a body of different sizes and forms in joint prosthesis, e.g. a hip joint ball, a hip joint socket, a knee joint wearing surface, etc.

3. A method according to claim 2 **characterised by** the usage of the biomaterial on one, both or all of the wearing surfaces in an artificial joint prosthesis.

4. A joint prosthesis, for example a hip or knee joint prosthesis, made of a ceramic material with a high stress tolerance and tissue tolerance for medical usage as a surface carrying orthopaedic biomaterial for the wearing surfaces in the artificial joint prosthesis,
**characterised in that** the material of the prosthesis body is a composition of a powder containing silicon nitride as a base, consisting of 70-100 volume % of silicon nitride with an addition of 0-30 volume % of one or a combination of the following materials: zirconium dioxide, yttrium dioxide, titanium dioxide, calcium oxide, silicon carbide and/or 0-20 volume % of short fibres of aluminium oxide, silicon carbide and mullite, or independent mixtures of these, whereupon the material mixture is formed to the joint prosthesis body in question and subjected to a high pressure of between 50 MPa and 350 MPa and temperatures of between 500-3000 °C by isostatic pressing and sintering (HIP) for a desired porosity of the body and wear surfaces of the body are polished for the wear to be minimized.

5. A prosthesis according to claim 4 **characterised in that** the body is formed, for example, as a hip joint head, a hip joint socket or a knee joint wearing surface.

6. A prosthesis according to claim 5 **characterised in that** one, both or all of the wearing surfaces are made of said ceramic powder material composition.

## Patentansprüche

1. Ein Verfahren zum Herstellen einer Gelenksprothese, z. B. einer Hüftgelenks- oder Knieprothese aus einem keramischen Material mit einer hohen Belastungstoleranz und Gewebetoleranz zur medizinischen Verwendung als einer Oberfläche, welche ein orthopädisches Biomaterial für die Verschleißoberflächen in der künstlichen Gelenksprothese trägt, **gekennzeichnet durch**
1.1. die Herstellung/Mischung einer Pulver Zusammensetzung, enthaltend Siliziumnitrid als einer Basis, bestehend aus 70-100 Volumen % Siliziumnitrid unter Zusatz von 0-30 Volumen % eines oder der Kombination der folgenden Materialien: Zirkondioxid, Yttriumdioxid, Titandioxid, Kalziumoxid, Siliziumcarbid und/oder 0-20 Volumen % kurze Fasern aus Aluminiumoxid, Siliziumcarbid und Mullit oder unabhängigen Mischungen dieser,
1.2. Formen des Gelenksprothesenkörpers aus der Mischung,
1.3 Hochdruckbehandeln des Prothesekörpers zwischen 50 MPa und 350 MPa und Temperaturen zwischen 500-3000°C **durch** isostatisches Pressen und Sintern (HIP), um eine erwünschte Porosität des Körpers zu erreichen, wonach
1.4. der Körper gekühlt wird und Verschleißoberflächen poliert werden, um den Verschleiß zu minimieren und vor der medizinischen Verwendung klinisch dekontaminiert wird.

2. Verfahren nach Anspruch 1, **gekennzeichnet durch** das Formen des Siliziumnitrids und seiner Mischungen zu einem Körper unterschiedlicher Größen und Formen in Gelenksprothesen, z. B. einer Hüftgelenkskugel, einer Hüftgelenkspfanne, einer Kniegelenksverschleißoberfläche usw.

3. Verfahren nach Anspruch 2, **gekennzeichnet durch** die Verwendung von Biomaterial auf einer, beiden oder sämtlichen Verschleißoberflächen in einer künstlichen Gelenksprothese.

4. Eine Gelenksprothese, z. B. eine Hüft- oder Kniegelenksprothese, hergestellt aus keramischem Material mit einer hohen Belastungstoleranz und Gewebstoleranz zur medizinischen Verwendung als eine Oberfläche, welche als die Verschleißoberflächen in der künstlichen Gelenksprothese orthopädisches Biomaterial trägt, **dadurch gekennzeichnet, daß** das Material des Prothesekörpers eine Zusammensetzung ist aus einem Pulver, enthaltend Siliziumnitrid als Basis, bestehend aus 70-100 Volumen % Siliziumnitrid unter Zusatz von 0-30 Volumen % eines oder einer Kombination der folgenden Materialien: Zirkondioxid, Yttriumdioxid, Titandioxid, Kalziumoxid, Siliziumcarbid und/oder 0-20 Volumen % kurzen Fasern aus Aluminiumoxid, Siliziumcarbid und Mullit oder unabhängige Mischungen dieser, woraufhin die Materialmischung zu dem in Rede stehenden Gelenksprothesenkörper geformt wird und einem Hochdruck zwischen 50 MPa und 350 MPa und Temperaturen zwischen 500-3000°C durch isostatisches Pressen und Sintern (HIP) ausgesetzt wird für eine gewünschte Porosität des Körpers, und die Verschleißoberflächen des Körpers poliert werden, um den Verschleiß zu minimieren.

5. Eine Prothese nach Anspruch 4, **dadurch gekennzeichnet, daß** der Körper ausgebildet wird, z. B. als ein Hüftgelenkskopf, eine Hüftgelenkspfanne oder eine Kniegelenksverschleißoberfläche.

6. Eine Prothese nach Anspruch 5, **dadurch gekennzeichnet, daß** eine, beide oder sämtliche Verschleißoberflächen aus der keramischen Pulvermaterialzusammensetzung hergestellt sind.

## Revendications

1. Procédé pour la fabrication d'une prothèse articulaire, par exemple une prothèse articulaire de la hanche ou du genou, à base d'un matériau céramique avec une tolérance à la contrainte et une tolérance tissulaire élevées pour usage médical à titre de surface de support, biomatériau orthopédique destiné aux surfaces de frottement pour les prothèses articulaires artificielles, **caractérisé en ce que**
1.1. la préparation/le mélange de la composition sous forme de poudre contenant des nitrures de silicium à titre de base consistant en 70-100% en volume de nitrure de silicium avec addition de 0-30% en volume de l'un ou de la combinaison des matériaux suivants : dioxyde de zirconium, dioxide d'yttrium, dioxide de titanium, oxyde de calcium, carbure de silicium et/ou 0-20% en volume de fibres courtes d'oxyde d'aluminium, de carbure de silicium et de mullite, ou des mélanges indépendants de ceux-ci ;
1.2 le moulage du corps de la prothèse articulaire à partir du mélange ;
1.3 la soumission du corps de la prothèse à de fortes pressions comprises entre 50 MPa et 350 MPa et à des températures comprises entre 500-3000°C par pressage isostatique et par frittage (HIP) de manière à ce que le corps acquière une porosité souhaitable ;
1.4 le corps est refroidi et les surfaces de frottement sont polies pour que le frottement soit minimisé et est décontaminé cliniquement avant usage médical.

2. Procédé selon la revendication 1, **caractérisé par** le moulage du nitrure de silicium et de ses mélanges en un corps de tailles et de formes différentes pour des prothèses, par exemple une sphère pour une articulation de la hanche, une surface de frottement pour une articulation du genou, etc.

3. Procédé selon la revendication 2, **caractérisé par** la mise en oeuvre du biomatériau sur l'une, deux ou toutes les surfaces de frottement d'une prothèse articulaire artificielle.

4. Prothèse articulaire, par exemple une prothèse articulaire de la hanche ou du genou, constituée de matériau céramique avec une tolérance à la contrainte et une tolérance tissulaire élevées pour un usage médical à titre de biomatériau orthopédique supportant une surface pour les surfaces de frottement des prothèses articulaires artificielles, **caractérisé en ce que** le matériau du corps de la prothèse est une composition sous forme de poudre contenant du nitrure de silicium en tant que base, consistant en 70-100% en volume de nitrure de silicium avec addition de 0-30% en volume de l'un ou d'une combinaison des matériaux suivants : dioxyde de zirconium, dioxyde d'yttrium, dioxyde de titanium, oxyde de calcium, carbure de silicium et/ou 0-20% en volume de fibres courtes d'oxyde d'aluminium, de carbure de silicium et de mullite, ou des mélanges indépendants de ceux-ci, dans lequel le mélange de matériaux est moulé en un corps pour la prothèse articulaire en question et soumis à une pression élevée comprise entre 50 MPa et 350 MPa et à des températures comprises entre 500-3000°C par pressage isostatique et frittage (HIP) pour une porosité souhaitable du corps et les surfaces de frottement du corps sont polies pour que le frottement soit minimisé.

5. Prothèse selon la revendication 4, **caractérisée en ce que** le corps est moulé, par exemple, sous forme d'une sphère pour une articulation de la hanche ou sous forme d'une surface de frottement pour une articulation du genou.

6. Prothèse selon la revendication 5, **caractérisée en ce qu'**une, deux ou toutes les surfaces de frottement sont constituées d'une composition de matériau céramique sous forme de poudre.
